# EUROPÄISCHE PATENTSCHRIFT

(11) **EP 0 873 984 B1**
(45) Veröffentlichungstag und Bekanntmachung des Hinweises auf die Patenterteilung: **12.09.2001**
(21) Anmeldenummer: 98103918.3
(22) Anmeldetag: 05.03.1998
(51) Int. Cl.: C07C 51/09, C07C 55/02

(54) **Verfahren zur Herstellung von Alkyl- und Arylmalonsäuren**
Process for preparing alkyl- and aryl- substituted malonic acids
Procédé de préparation d'acides maloniques alkyl et aryl substitués

(30) Priorität: 21.04.1997 DE 19716615
(43) Veröffentlichungstag der Anmeldung: 28.10.1998
(73) Patentinhaber: Degussa AG, 40474 Düsseldorf (DE)
(72) Erfinder: Steffen, Klaus-Dieter, Dr., 53773 Hennef (DE)

(56) Entgegenhaltungen:
- DE-A- 4 120 704

## Beschreibung

Die Erfindung betrifft ein neues Verfahren zur Herstellung von Alkyl- und Arylmalonsäuren der Formel I 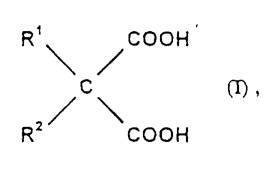 wobei
- R¹ =: H, C₁- bis C₁₂-Alkyl, Phenyl, C₁- bis C₄-Alkylphenyl, C₂- bis C₄-Dialkylphenyl,
- R² =: C₁- bis C₁₂-Alkyl, Phenyl, C₁- bis C₄-Alkylphenyl, C₂- bis C₄-Dialkylphenyl oder
- R¹ + R² =: -CH₂-CH₂- ist,
durch alkalische Verseifung der entsprechenden C₁- bis C₄-Alkylester.

Alkyl- und Arylmalonsäuren sind wichtige Zwischenprodukte für die Synthese von Agrochemikalien und Pharmawirkstoffen. Sie werden beispielsweise zur Herstellung von "Meldrum's Säuren", Barbituraten, Riechstoffen und Vitaminen eingesetzt.

Die Herstellung von substituierten Malonsäuren wird allgemein in Kirk-Othmer, Encyclopedia of Chemical Technology, 3. Ed. (1981), Vol. 14, 794 bis 810, angegeben. Es kann eine saure oder alkalische Verseifung von Carbonsäureestern, -nitrilen oder -amiden angewandt werden. Dabei ist es oft ein Problem, daß die Produkte in Mischungen mit Natriumchlorid anfallen.

Erschwerend bei der Herstellung der substituierten Malonsäuren ist ihre sehr gute Löslichkeit in Wasser und ihre leichte Decarboxylierung, die bei der Malonsäure bereits ab 70 °C einsetzt.

Die bekannteste Herstellung von Methylmalonsäure geht von α-Chlorpropionsäure aus, die mit Natriumcyanid zu dem entsprechenden Nitril umgesetzt und dann mit Natronlauge unter NH₃-Abspaltung verseift wird. Es folgt gemäß Org. Syntheses, Col. Vol. II (1943), 376, eine umständliche Isolierung über die Ca-Salze.

Wenn nach der alkalischen Verseifung eines Malonesters die Malonsäure als Alkalisalz gelöst in Wasser vorliegt, können die Alkali-Kationen nach DE-A-41 20 704 über saure Ionenaustauscher entfernt und anschließend die freie Säure isoliert werden. Da bei alkalischen Verseifungen stets mindestens stöchiometrische Mengen an Alkali eingesetzt werden müssen, die bei der Malonsäure-Aufarbeitung wieder mit starken Säuren neutralisiert werden, fallen bei dieser Methode mindestens stöchiometrische Mengen an Salz an, die oft aufwendig entsorgt werden müssen. Das ist ein großer Nachteil dieser Methode der alkalischen Verseifung.

Cyclopropan-1,1-dicarbonsäure kann nach Org. Syntheses, Vol. 60 (1981), 66, aus Diethylmalonat, 1,2-Dibromethan, Natronlauge und stöchiometrischen Mengen eines Phasentransfer-Katalysators durch gleichzeitige Verseifung des intermediär gebildeten Cyclopropan-1,1-dicarbonsäurediethylesters in ca. 70 %iger Ausbeute hergestellt werden. Neben der großen Menge an Phasentransfer-Katalysator, der 2,5fachen Menge, bezogen auf die erhaltene Cyclopropan-1,1-dicarbonsäure, fallen auch beträchtliche Mengen an Natriumchlorid an, die - in Wasser gelöst - entsorgt werden müssen.

Dimethylmalonsäure ist ebenfalls lange bekannt und kann durch alkalische Verseifung des entsprechenden Diethylesters hergestellt werden. Die Verseitung des Dimethylesters mit Kalilauge zur Säure wird von W. Schauzer, K. Clusius, Z. physik. Chemie A 190 (1941), 243, ohne Nennung von Ausbeute und Reinheit erwähnt. Nach anderen Methoden wird die Säure durch Oxidation mit KMnO₄ oder HNO₃ von Methyl-Vorstufen gewonnen.

Bei allen diesen bekannten Verfahren zur Herstellung der substituierten Malonsäuren durch alkalische Verseifung der entsprechenden Ester fällt, bezogen auf das Produkt, die 2fach-molare Menge an in Wasser gelöstem Salz an. Dabei müssen diese Salzlösungen entsorgt werden, was zu einem Problem bei der biologischen Abwasserreinigung und zu Schwierigkeiten mit behördlichen Vorschriften führt. Ein anderer Weg, das Abdampfen des Wassers, erfordert große Energiemengen.

Aufgabe der vorliegenden Erfindung war es daher, ein Verfahren bereitzustellen, nach dem die bei der alkalischen Hydrolyse zwangsläufig entstehenden Salze in einfacher Form ohne hohen Energieaufwand abgetrennt und isoliert werden können.

Die Aufgabe wird erfindungsgemäß dadurch gelöst, daß man
- die Hydrolyse durch Alkalihydroxid, gelöst in einer wäßrigen, zu 90 bis 100 % gesättigten Alkalisalz-Lösung, vornimmt,
- nach der Hydrolyse die Lösung mit einer Mineralsäure ansäuert,
- ausgefälltes Alkalisalz abtrennt und
- die entstandenen Alkyl- und Arylmalonsäuren aus der wäßrigen Lösung mit einem organischen Lösemittel extrahiert.

Geeignete Substituenten R¹ und R² gemäß Formel I sind beispielsweise Methyl, Ethyl, Isopropyl, n-Butyl, n-Octyl sowie Methyl-, Ethyl- oder Dimethylphenyl.

Als C₁- bis C₄-Ester kommen beispielsweise die Methyl-, Ethyl-, Isopropyl- oder n-Butylester in Betracht. Vorzugsweise werden die Methylester eingesetzt.

Geeignete Alkalihydroxide sind beispielsweise Natrium-, Kalium-, Lithium- oder Caesiumhydroxid. Vorzugsweise verwendet man Natrium- oder Kaliumhydroxid Das Alkalihydroxid wird meist in Mengen von 2 bis 4 Mol pro Mol substituiertem Malonsäurediester eingesetzt. Vorzugsweise wird ein Alkalihydroxid-Überschuß von 1 bis 50 Mol-% angewandt.

KOH wird ganz besonders bevorzugt, da die Kaliumsalze oft die geringere Löslichkeit gegenüber den Natriumsalzen besitzen.

Vorzugsweise wird das Alkalihydroxid in einer wäßrigen gesättigten Alkalisalz-Lösung gelöst.

Als Mineralsäuren, die hier eingesetzt werden können, werden beispielsweise Schwefel-, Salz-, Phosphor-, Jodwasserstoff-, Chlor- und Perchlorsäure genannt. Vorzugsweise wird mit Schwefelsäure oder Salzsäure angesäuert.

Die nach der Hydrolyse vorliegende wäßrige Lösung des Alkalisalzes der substituierten Malonsäure wird mit der Mineralsäure im allgemeinen bis auf einen pH-Wert von 1 bis 2 angesäuert. Dabei liegt dann die Alkyl- oder Arylmalonsäure vollständig als freie Säure vor. Bezogen auf den ursprünglich eingesetzten Diester werden meist 2 bis 4 Mol Mineralsäure eingesetzt, wobei man vorzugsweise einen Säureüberschuß von 1 bis 50 Mol-% verwendet.

Vorzugsweise werden Alkalihydroxid und Alkalisalz-Lösung des gleichen Alkalimetalls angewandt, wobei beim Ansäuern die Mineralsäure eingesetzt wird, die dem Anion des Alkalisalzes entspricht. Besonders bevorzugt werden KOH als Alkalihydroxid, K₂SO₄ als Alkalisalz und H₂SO₄ als Mineralsäure eingesetzt.

Die Abtrennung des ausgefällten oder auskristallisierten Alkalisalzes kann durch Filtration oder Zentrifugieren erfolgen, wobei die Filtration bevorzugt wird. Die Alkylund Arylmalonsäuren sind dabei je nach Substituent mehr oder weniger wasserlöslich. Sie können schon allein wegen des "Aussalzeffektes" teilweise mit auskristallisieren. Sie können aber schnell wieder in Lösung gebracht werden, wenn man den abgetrennten Feststoff mit einem Alkohol wäscht.

Als Extraktionsmittel eignen sich allgemein mit Wasser wenig mischbare organische Lösemittel, die ausreichend polar sind, um die substituierten Malonsäuren aufzunehmen. Geeignet sind beispielsweise Ketone, langkettige Alkohole oder Ether, wobei Dialkylether mit 3 bis 8 C-Atomen bevorzugt werden. Beispiele dafür sind Methylethylether, Diethylether, Methyl-t-butylether, Diisopropylether und Dibutylether, wobei die beiden letztgenannten besonders bevorzugt eingesetzt werden.

Durch die hohen Salzkonzentrationen in der wäßrigen Phase wird die Extraktion stark erleichtert. Die Extraktion kann kontinuierlich z. B. mittels einer Mixer-Settler-Apparatur oder diskontinuierlich durch 2- oder 3maliges Ausschütteln bzw. Ausrühren erfolgen.

Nach der Extraktion wird das Lösemittel ggf. nach Trocknung eingedampft. Dabei fällt die Alkyl- oder Arylmalonsäure kristallin an. Man kann auch auf die Trocknung verzichten, da mit dem Abdampfvorgang meist auch eine azeotrope Trocknung verbunden ist.

Es kann auch vorteilhaft sein, gegen Ende des Abdampfvorganges ein organisches Nichtlösemittel für die Alkyl- oder Arylmalonsäure zuzusetzen, wodurch das Produkt ausgefällt wird und abfiltriert werden kann. Durch Waschen und Trocknen kann so oft ein besonders sauberes Produkt erhalten werden.

Nach dem erfindungsgemäßen Verfahren können die auskristallisierten Alkalisalze durch einfache Filtration isoliert werden. Sie fallen dabei in einer ausreichend reinen Form an, um anderweitig verwendet werden zu können. Nach Waschung mit einem Alkohol, vorzugsweise mit dem Alkohol, der bei der Hydrolyse entsteht, und Trocknung kann z. B. K₂SO₄ als Düngemittel eingesetzt oder für die Herstellung von Alaun, Persulfat, Pottasche und andere Produkte verwendet werden.

Nach der Extraktion der Alkyl- und Arylmalonsäuren können die wäßrigen Lösungen auch beliebig oft für neue Ansätze wiederverwendet werden.

Das Verfahren ist vorteilhaft anwendbar für die Herstellung von Mono- und Dialkylmalonsäuren und besonders für die Synthese von Phenylmalonsäure, da das eine für die Synthese von Phenylmalonsäuredialkylester notwendige Äquivalent an Alkali (in Form des Alkalialkoholats) auch für die Hydrolyse des Esters ausgenutzt werden kann.

Für das Verfahren können auch die Halbester der Alkyl- und Arylmalonsäuren eingesetzt werden.

Die folgenden Beispiele sollen die Erfindung verdeutlichen.

### Beispiel 1

### Herstellung von Diethylmalonsäure (DEMS)

In einem 2-1-Mehrhalskolben mit Rührer, Thermometer, Tropftrichter und Destillationsteil werden eingewogen: 530 ml wäßrige gesättigte K₂SO₄-Lösung (10 %ig, aus vorhergehenden Ansätzen), 206 g KOH-Schuppen (85 %ig, 3,12 Mol) und 500 ml Ethanol 95 %ig (Rückgewinnung aus vorhergehenden Ansätzen). Dann wird zum Sieden erhitzt, bis eine klare Lösung vorliegt. In 1 h werden 270,5 g Diethylmalonsäurediethylester (1,25 Mol) zugetropft und in 5 bis 6 Stunden 160 bis 180 ml Ethanol abdestilliert. Der Ansatz wird abgekühlt. Dann werden unter Kühlung (bis ca. 25 °C) 177 g konz. H₂SO₄ (96 %ig, 1,73 Mol), die vorher mit ca. der gleichen Menge an H₂O verdünnt werden, zudosiert, bis der pH-Wert auf 1 bis 2 abgefallen ist.

Das auskristallisierte K₂SO₄ wird abfiltriert, 2mal mit Ethanol gewaschen und getrocknet: 272 g (Ausbeute: 100 % d. Th.).

Von dem Filtrat wird unter partiellem Vakuum von ca. 300 mbar das gesamte Ethanol abdestilliert und aus der wäßrigen Lösung die DEMS durch 3maliges Ausschütteln mit Methyl-t-butylether (400 ml, 200 ml, 100 ml) extrahiert.

Methyl-t-butylether wird weitgehend abdestilliert und durch Toluol ersetzt (400 ml). Alle Reste an Wasser und Methyl-t-butylether werden destillativ entfernt Die auskristallisierte DEMS wird abfiltriert, mit Toluol gewaschen und im Vakuum getrocknet.

| | |
|---|---|
| Auswaage | 190 g (95 % d. Th.) |
| Reinheit | 99,4 % (Titration, GC) |
| Schmelzpunkt | 120 °C |

Alle Lösemittel-Destillate und die wäßrige gesättigte K₂SO₄-Lösung (nach Neutralisation mit KOH) werden bei den Folgeansätzen wiederverwendet.

### Beispiel 2

### Herstellung von Ethylmalonsäure (EMS)

In einen 2-l-Mehrhalskolben, versehen mit Rührer, Thermometer, Tropftrichter und Destillationsaufsatz, werden eingefüllt: 600 g wäßrige, mit K₂SO₄ gesättigte Lösung aus einem vorherigen Ansatz, 203 g KOH-Schuppen (87 %ig, 3,15 Mol) und 350 ml Ethanol (Rückgewinnung aus vorherigem Ansatz).

Es wird unter Rühren aufgeheizt, bis alles gelöst ist, dann werden 282,1 g Ethylmalonsäurediethylester (1,5 Mol) in 90 Minuten bei 70 bis 80 °C zudosiert. Während der Nachreaktionszeit von 2 bis 3 Stunden wird ein Teil des Alkohols (ca. 200 g) abdestilliert, dann die wäßrige Reaktionslösung unter Kühlung mit 168,8 g H₂SO₄ (96 %ig, 1,65 Mol), die vorher mit 100 ml Wasser verdünnt worden ist, angesäuert. Das auskristallisierte K₂SO₄ wird abfiltriert, mit Ethanol gewaschen und getrocknet: 274 g (Ausbeute: 99,8 % d. Th.). Von den gesammelten Filtraten wird der gesamte Alkohol unter partiellem Vakuum von 150 bis 300 mbar abdestilliert, dann die verbleibende wäßrige Lösung 4mal mit Methyl-t-butylether (400 ml, 200 ml, 200 ml, 100 ml) extrahiert.

Die organischen Extrakte werden vereinigt und der gesamte Methyl-t-butylether abdestilliert, wobei EMS trocken auskristallisiert.

| | |
|---|---|
| Auswaage | 191 g (96,4 % d. Th.) |
| Reinheit | 99,4 % (Titration, GC) |
| Schmelzpunkt | 111 °C |

Das wäßrige K₂SO₄-haltige Filtrat wird mit KOH neutral gestellt und bei Folgeansätzen für die Hydrolyse eingesetzt.

### Beispiel 3

### Herstellung von Cyclopropan-1,1-dicarbonsäure (CDS)

In einem 4-l-Mehrhalskolben werden 800 ml wäßrige gesättigte K₂SO₄-Lösung (aus vorhergehenden Ansätzen, neutral gestellt), 500 ml Methanol und 330 g KOH (85 %ig, 5,0 Mol) erhitzt, worauf bei 68 °C innerhalb von 90 Minuten 316,2 g Cyclopropan-1,1-dicarbonsäuredimethylester (2,0 Mol) zudosiert werden. Während der Nachreaktionszeit von 4 Stunden wird ein Teil des Methanols abdestilliert. Nach dem Abkühlen der Reaktionslösung wird mit 281 g Schwefelsäure (96 %ig, 2,75 Mol), die vorher mit 100 ml Wasser verdünnt worden ist, unter Kühlung angesäuert. Das auskristallisierte K₂SO₄ wird über eine Fritte abfiltriert, mit Methanol gewaschen und getrocknet: 380 g (87,2 % d. Th.).

Von den gesammelten Filtraten wird alles Methanol in partiellem Vakuum abdestilliert, worauf die wäßrige Lösung 2mal mit Diisopropylether (400 ml, je 200 ml) extrahiert wird. Die vereinten Etherphasen werden zur Entfernung von K₂SO₄-Resten kurz mit 30 ml Wasser gewaschen, dann im Vakuum zur Trockene eingedampft, wobei die CDS kristallin ausfällt.

| | |
|---|---|
| Auswaage | 216,9 g (83,4 % d.Th.) |
| Reinheit | 98,8 % (Titration, GC) |
| Schmelzpunkt | 136 - 137,5 °C |

Die Destillate der organischen Lösemittel und das wäßrige K₂SO ₄Filtrat (nach Neutralisation mit KOH) werden bei den Folgeansätzen wieder eingesetzt.

### Beispiel 4

### Herstellung von Phenylmalonsäure (PMS)

In einem 2-1-Mehrhalskolben mit Destillationsteil werden 300 g einer 32 %igen Kaliummethylat-Lösung (1,37 Mol) weitgehend zur Trockene eingeengt. Auf die Kaliummethylat-Kristalle werden 205,5 g Ethylphenyl-acetat (1,25 Mol) und 826 g Diethylcarbonat (7,0 Mol, frisch und Rückgewinnung) gegeben, worauf bei ca. 500 mbar Vakuum sowie ca. 90 °C Sumpftemperatur das gesamte Methanol und Ethanol abdestilliert wird. Nach Reaktionsende wird auch das überschüssige Diethylcarbonat im partiellen Vakuum abdestilliert, bis nur ein K-Salz von Phenylmalonsäurediethylester übrig bleibt.

Dieses Salz wird in ca. 500 ml einer wäßrigen gesättigten K₂SO₄-Lösung (Filtrat aus vorhergehenden Ansätzen) gelöst und mit einer konz. KOH-Lösung (1,54 g KOH, 85 %ig, 2,33 Mol) versetzt. Es folgt die Verseifung über 4 bis 5 Stunden bei ca. 90 °C.

Nach dem Abkühlen wird vorsichtig mit 201 g konz. H₂SO₄ (96 %ig, 1,97 Mol), die vorher mit H₂O verdünnt worden ist, auf einen pH-Wert von ca. 1,5 angesäuert. Dabei fällt K₂SO₄ und ein Teil der PMS aus. Aufarbeitung:

Die PMS wird durch Zusatz von Alkohol wieder in Lösung gebracht, K₂SO₄ abfiltriert, gewaschen und getrocknet: 322 g (100 % d. Th.). Aus dem Filtrat wird nach Abdestillation des Alkohols die PMS durch 3malige Extraktion mit Methyl-t-butylether (800 ml) gewonnen. (Man kann PMS auch direkt aus der Salz-Suspension durch Extraktion mit Methyl-t-butylether gewinnen und anschließend die K₂SO₄-Kristalle abfiltrieren.)

Die gesammelten Methyl-t-butylether-Extrakte werden zur Trockene eingeengt, wobei PMS-Kristalle ausfallen, die zur Reinigung nochmals in Toluol aufgenommen werden und dann abfiltriert werden können.

| | |
|---|---|
| Auswaage | 189 g (84 % d. Th.) |
| Schmelzpunkt | 152-153 °C (Zers.) |
| Reinheit | 99,7 % (Titration, Ionenchrom.) |

Die Herstellung von PMS kann ebenso gut mit Kaliumethylat durchgeführt werden. Dann fällt nur Ethanol als Destillat an; die PMS-Kristalle können einen Gelbstich annehmen.

Die extrahierten, K₂SO₄-gesättigten Phasen und Methyl-t-butylether-Destillate sind für Folgeansätze wieder einsetzbar.

## Patentansprüche

1. Verfahren zur Herstellung von Alkyl- und Arylmalonsäuren der Formel I 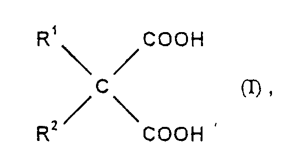 wobei
R¹ = H, C₁- bis C₁₂-Alkyl, Phenyl, C₁- bis C₄-Alkylphenyl, C₂- bis C₄-Dialkylphenyl
R² = C₁-bis C₁₂-Alkyl, Phenyl, C₁- bis C₄-Alkylphenyl, C₂- bis C₄-Dialkylphenyl oder
R¹ + R² = -CH₂-CH₂- ist,
durch alkalische Verseifung der entsprechenden C₁- bis C₄-Alkylester,
**dadurch gekennzeichnet**,
- daß man mit Alkalihydroxid, gelöst in einer wäßrigen, zu 90 bis 100 % gesättigten Alkalisalz-Lösung, hydrolysiert,
- nach der Hydrolyse mit einer Mineralsäure ansäuert,
- ausgefälltes Alkalisalz abtrennt und
- die gebildeten Alkyl- und Arylmalonsäuren aus der wäßrigen Lösung mit einem organischen Lösemittel extrahiert.

2. Verfahren nach Anspruch 1,
**dadurch gekennzeichnet,**
**daß** man mit Natrium- oder Kaliumhydroxid hydrolysiert.

3. Verfahren nach Anspruch 1,
**dadurch gekennzeichnet,**
**daß** man das Alkalihydroxid in einer wäßrigen gesättigten Alkalisalz-Lösung löst.

4. Verfahren nach Anspruch 1,
**dadurch gekennzeichnet,**
**daß** man mit Salz- oder Schwefelsäure ansäuert.

5. Verfahren nach Anspruch 1,
**dadurch gekennzeichnet,**
**daß** man mit KOH in einer K₂SO₄-Lösung hydrolysiert und mit H₂SO₄ ansäuert.

6. Verfahren nach Anspruch 1,
**dadurch gekennzeichnet,**
**daß** man mit einem Dialkylether extrahiert.

7. Verfahren nach Anspruch 6,
**dadurch gekennzeichnet,**
**daß** man mit Methyl-t-butylether oder Diisopropylether extrahiert.

## Claims

1. A process for preparing alkyl- and arylmalonic acids of the formula I 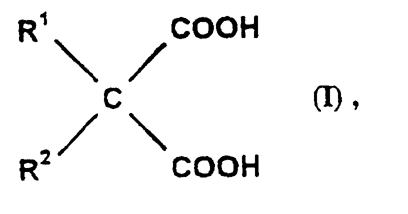 where
R¹ = H, C₁-C₁₂-alkyl, phenyl, C₁-C₄-alkylphenyl, C₂-C₄-dialkylphenyl,
R² = C₁-C₁₂-alkyl, phenyl, C₁-C₄-alkylphenyl, C₂-C₄-dialkylphenyl or
R¹ + R² = -CH₂-CH₂-,
by alkaline saponification of the corresponding C₁-C₄-alkyl esters,
**characterized in that**
- the hydrolysis is effected with alkali metal hydroxide, dissolved in an aqueous alkali metal salt solution saturated to from 90 to 100%,
- hydrolysis is followed by acidification with a mineral acid,
- precipitated alkali metal salt is removed and
- the alkyl- and arylmalonic acids formed are extracted from the aqueous solution with the aid of an organic solvent.

2. A process according to claim 1,
**characterized in that**
the hydrolysis is effected with sodium hydroxide or potassium hydroxide.

3. A process according to claim 1,
**characterized in that**
the alkali metal hydroxide is dissolved in an aqueous saturated alkali metal salt solution.

4. A process according to claim 1,
**characterized in that**
acidification is effected with hydrochloric acid or sulphuric acid.

5. A process according to claim 1,
**characterized in that**
hydrolysis is effected with KOH in a K₂SO₄ solution, followed by acidification with H₂SO₄.

6. A process according to claim 1,
**characterized in that**
extraction takes place with the aid of a dialkyl ether.

7. A process according to claim 6,
**characterized in that**
extraction takes place with the aid of methyl t-butyl ether or diisopropyl ether.

## Revendications

1. Procédé de préparation d'acides alkyl et arylmaloniques de formule I, 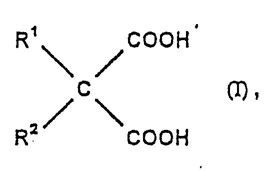 dans laquelle
R¹ = H, un alkyle en C₁ à C₁₂, un phényle, un (alkyle en C₁ à C₄)phényle, un (dialkyl en C₂-C₄)phényle,
R² = un alkyle en C₁ à C₁₂), un phényle, un (alkyle en C₁ à C₄)phényle, un (dialkyl en C₂-C₄)phényle ou,
R¹ + R² = -CH₂CH₂-,
par saponification en milieu alcalin de l'ester d'alkyle en C₁-C₄ correspondant,
**caractérisé en ce qu'**
- on hydrolyse avec un hydroxyde de métal alcalin dissout dans une solution aqueuse, saturée de 90 à 100 % de sel de métal alcalin,
- on acidifie après l'hydrolyse avec un acide minéral,
- on sépare le sel de métal alcalin qui a précipité, et
- les acides alkyl et arylmaloniques formés sont extraits de la solution aqueuse avec un solvant organique.

2. Procédé selon la revendication 1,
**caractérisé en ce qu'**
on hydrolyse avec de l'hydroxyde de sodium ou de potassium.

3. Procédé selon la revendication 1,
**caractérisé en ce qu'**
on dissout l'hydroxyde de métal alcalin dans une solution aqueuse saturée de sel de métal alcalin.

4. Procédé selon la revendication 1,
**caractérisé en ce qu'**
on acidifie avec de l'acide chlorhydrique ou de l'acide sulfurique.

5. Procédé selon la revendication 1,
**caractérisé en ce qu'**
on hydrolyse avec KOH dans une solution de K₂SO₄ et on acidifie avec H₂SO₄.

6. Procédé selon la revendication 1,
**caractérisé en ce qu'**
on extrait avec un éther dialkylique.

7. Procédé selon la revendication 6,
**caractérisé en ce qu'**
on extrait avec de l'éther méthyl-ter-butylique ou de l'éther diisopropylique.
